Europäisches Patentamt

European Patent Office

Office européen des brevets

(19)

(11) **EP 1 165 717 B1**

(12) **FASCICULE DE BREVET EUROPEEN**

(45) Date de publication et mention
de la délivrance du brevet:
**08.06.2005 Bulletin 2005/23**

(21) Numéro de dépôt: **00910912.5**

(22) Date de dépôt: **09.03.2000**

(51) Int Cl.[7]: **C09J 153/02**, A61L 15/58

(86) Numéro de dépôt international:
**PCT/FR2000/000582**

(87) Numéro de publication internationale:
**WO 2000/053690 (14.09.2000 Gazette 2000/37)**

(54) **NOUVELLE MASSE ADHESIVE HYDROCOLLOIDE PRESENTANT UNE MEILLEURE RESISTANCE A LA DEGRADATION DE SA CAPACITE D'ABSORPTION APRES RADIOSTERILISATION**

HYDROKOLLOIDKLEBSTOFFMASSE MIT EINEM VERBESSERTEN WIDERSTAND ZU ABSORPTIONDEGRADIERUNGSKAPAZITÄT NACH RADIOSTERILISATION

NOVEL HYDROCOLLOID ADHESIVE MASS WITH IMPROVED RESISTANCE TO DETERIORATION OF ITS ABSORPTION CAPACITY AFTER BEING STERILISED BY RADIATION

(84) Etats contractants désignés:
**AT BE CH CY DE DK ES FI FR GB GR IE IT LI LU MC NL PT SE**

(30) Priorité: **09.03.1999 FR 9902870**

(43) Date de publication de la demande:
**02.01.2002 Bulletin 2002/01**

(73) Titulaire: **Holding Urgo Participations - HUP
21300 Chenove (FR)**

(72) Inventeurs:
• **AUGUSTE, Stéphane
  F-21800 Quetigny (FR)**

• **APERT, Laurent
  F-21000 Dijon (FR)**
• **GARIMA, Luc
  F-21000 Dijon (FR)**

(74) Mandataire: **Hubert, Philippe
  Cabinet Beau de Loménie
  158, rue de l'Université
  75340 Paris Cédex 07 (FR)**

(56) Documents cités:
  **EP-A- 0 730 874          WO-A-98/10801
  WO-A-99/45977             DE-A- 4 207 657
  US-A- 4 231 369           US-A- 4 738 257
  US-A- 5 456 745**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention a pour objet de nouvelles masses adhésives hydrocolloïdes présentant une meilleure résistance à la dégradation de leur capacité d'absorption après radiostérilisation.

Plus précisément la présente invention a pour objet de nouvelles masses adhésives hydrocolloïdes constituées d'un mélange adhésif, à base d'un polyisobutylène de bas poids moléculaire et d'un polymère séquencé poly(styrène-oléfine-styrène), et d'un hydrocolloïde dérivé de cellulose, auxquels est associé un polymère acrylate ayant une température de transition vitreuse inférieure à - 20 °C dans le but d'augmenter la résistance à la dégradation de la capacité d'absorption après radiostérilisation desdites masses adhésives hydrocolloïdes.

L'invention concerne également l'utilisation de ces nouvelles masses adhésives hydrocolloïdes à des fins médicales, dermatologiques ou cosmétiques en particulier pour la réalisation de pansements pour le traitement de l'ampoule, des plaies exsudatives, brûlures et des lésions dermo-épidermiques superficielles, profondes, chroniques ou aiguës.

Les masses adhésives hydrocolloïdes à base de polyisobutylène, de copolymère séquencé poly(styrène-oléfine-styrène) ou d'un mélange de ces deux polymères sont connues depuis longtemps. De telles masses adhésives hydrocolloïdes sont par exemple décrites dans les brevets US 3 339 546, US 4 231 369 ou US 4 551 490. Ces masses adhésives hydrocolloïdes sont employées dans de nombreuses applications médicales comme par exemple les dispositifs d'ostomies et pour la réalisation de pansements pour le traitement de l'ampoule, des plaies exsudatives, brûlures et des lésions dermo-épidermiques superficielles, profondes, chroniques ou aiguës.

Le document WO 99/45977, opposable au titre de l'article 54(3) CBE, décrit des masses adhésives hydrocolloïdes notamment à base de polymère tribloc de type poly(styrène-oléfine-styrène) comportant un ester d'acide gras de sorbitan éthoxylé dans le but d'obtenir une capacité d'absorption accrue lors des premières heures d'utilisation.

Afin d'être utilisés sans risque de contamination par des micro-organismes tous ces produits et en particulier les pansements cicatrisants doivent être impérativement stériles.

Différentes techniques existent pour détruire les micro-organismes contaminants telles la stérilisation par vapeur saturante ou par chaleur sèche, la stérilisation par gaz (oxyde d'éthylène, formaldéhyde) ou la stérilisation par radiation.

Toutefois toutes ne conviennent pas pour la réalisation de produits notamment ceux ayant des applications pharmaceutiques et en particulier des produits contenant des masses adhésives hydrocolloïdes.

Ainsi la stérilisation par vapeur saturante ou par chaleur sèche n'est pas utilisable car la masse adhésive et l'hydrocolloïde supportent mal les hautes températures.

De même la stérilisation par gaz est en général évitée en raison des risques inhérents à la présence de gaz résiduels dans les pansements. De plus, cette technique ne permet pas d'obtenir une pénétration de l'agent stérilisant dans l'entier volume de la masse adhésive hydrocolloïde ce qui en limite l'efficacité.

La technique généralement utilisée pour la stérilisation de masses adhésives hydrocolloïdes est par conséquent la stérilisation par radiation qui permet d'assurer une stérilisation au coeur du produit donc très efficace. Deux types de rayonnements peuvent être utilisés à cet effet, à savoir les rayonnements β et γ. La dose stérilisante est ajustée en fonction de la biocharge initiale, c'est à dire de la quantité de germes présents avant stérilisation.

Ces radiations ionisantes provoquent la cassure de la double hélice de l'ADN des bactéries, rendues ainsi incapables de se reproduire, et permettent ainsi d'obtenir des produits stériles.

Afin d'assurer une décontamination efficace avec une marge de sécurité suffisante, on applique généralement aux produits à stériliser une dose moyenne de 25 kGray. En pratique un produit reçoit une dose qui varie entre 25 et 45 suivant le procédé utilisé.

Cependant, ces deux techniques de radiostérilisation connues ont aussi des effets indésirables sur les masses adhésives hydrocolloïdes traitées. En particulier, ces rayons sont assez puissants pour casser les liaisons carbone-carbone et carbone-hydrogène des polymères adhésifs employés et provoquent alors des ruptures de chaînes dans ces macromolécules et des réductions de leur masse moléculaire moyenne qui influent sur leurs propriétés en particulier cohésives.

Dans le cas de masses adhésives à base de polyisobutylène de tels effets secondaires sont bien connus et par exemple résumés dans « Advances in Pressure Sensitive Adhesive Technology-2 » édité par Donatas Satas en avril 95 dans le chapitre 7 « Wound dressing » en particulier pages 165-166.

Ainsi l'action négative de l'irradiation sur la cohésion de masses adhésives hydrocolloïdes conduit, en particulier lors de l'utilisation du produit qui gonfle par absorption des liquides et exsudats, à des phénomènes de fluage de l'adhésif et au démantèlement ou la désintégration du produit.

Une des solutions qui a été préconisée pour éviter ce problème est l'ajout d'un composé destiné à réticuler ladite masse adhésive et ainsi renforcer son intégrité. Ainsi, le brevet US 4738257 décrit l'ajout d'un copolymère éthylène-acétate de vinyle au polyisobutylène qui permet de réticuler la masse lors de l'irradiation γ. L'utilisation d'un mélange d'un polyisobutylène de haut poids moléculaire et d'un polyisobutylène de bas poids moléculaire ou l'addition d'un polymère séquencé poly(styrène-isoprène-styrène) ou poly(styrène-butadiène-styrène) au polyisobutylène ont également été préconisées pour résoudre le problème précité.

Toutefois cet état de la technique est muet sur un autre effet indésirable résultant de la radiostérilisation des masses adhésives hydrocolloïdes.

On a en effet constaté que les masses adhésives à base d'un mélange polyisobutylène-polymère séquencé poly(styrène-oléfine-styrène) qui contiennent un dérivé de cellulose comme par exemple la carboxyméthylcellulose de sodium, subissent une baisse importante de leur capacité d'absorption après radiostérilisation. Cette perte d'absorption non véritablement expliquée pourrait résulter de la dégradation du réseau macromoléculaire du dérivé de cellulose par les rayonnements. La diminution importante de la capacité d'absorption après stérilisation, de ces masses adhésives hydrocolloïdes et des produits les contenant, apparaît tout aussi préjudiciable que la diminution de cohésion, dans la mesure où elle touche également une propriété essentielle de ces produits.

La réalisation d'une masse adhésive hydrocolloïde constituée d'un polyisobutylène, d'un copolymère séquencé poly(styrène-oléfine-styrène) et en tant qu'hydrocolloïde d'un dérivé de cellulose, qui présenterait une meilleure stabilité ou résistance à la dégradation de sa capacité d'absorption après irradiation, constituerait donc une amélioration considérable vis à vis de l'état de la technique existant.

Il a été découvert et ceci constitue le fondement de la présente invention que cette perte de la capacité d'absorption après stérilisation de telles masses adhésives hydrocolloïdes peut être diminuée de façon significative par l'incorporation dans ces masses adhésives hydrocolloïdes d'un polymère acrylate ayant une température de transition vitreuse inférieure à - 20 °C.

Quoique le mode d'action (protection du dérivé de cellulose, modification des phases du mélange ou autre) par lequel ce polymère acrylate permet de diminuer la dégradation de la capacité d'absorption après irradiation de ces masses adhésives hydrocolloïdes ne soit ni connu, ni expliqué, les inventeurs ont mis en évidence des résultats remarquables.

L'utilisation d'un tel polymère acrylate ayant une température de transition vitreuse inférieure à - 20 °C pour augmenter le pouvoir d'absorption d'une masse adhésive hydrocolloïde à base de copolymère séquencé poly(styrène-oléfine-styrène) a été décrite dans la demande de brevet WO 98/10801. Toutefois le problème de la stérilisation des produits n'est jamais envisagé dans ce document antérieur et l'homme de métier ne pouvait y discerner une quelconque information utile quant à l'aptitude de ce polymère acrylate à augmenter de façon significative la résistance à la dégradation de la capacité d'absorption après radiostérilisation d'une masse adhésive hydrocolloïde telle que décrite dans ledit document et a fortiori d'une masse adhésive à base de polyisobutylène et de copolymère séquencé poly(styrène-oléfine-styrène) dans laquelle l'hydrocolloïde est en outre spécifiquement constitué d'un dérivé de cellulose.

## Objets de l'invention

Ainsi, selon un premier aspect, la présente invention a pour objet une masse adhésive hydrocolloïde notamment utilisable à des fins médicales, caractérisée en ce que ladite masse adhésive hydrocolloïde comprend :

(a) 2 à 15 parties en poids d'un polymère acrylate ayant une température de transition vitreuse inférieure à - 20 °C,
(b) 20 à 50 parties en poids d'au moins un dérivé de cellulose,
(c) 32 à 120 parties en poids d'un mélange adhésif constitué d'au moins un polyisobutylène de bas poids moléculaire et d'un polymère séquencé poly (styrène-oléfine-styrène) auxquels sont associés un ou plusieurs composés choisis parmi les polyisobutylènes de haut poids moléculaire, les polybutènes, les résines poisseuses dites tackifiantes, les caoutchoucs butyle, les plastifiants et les antioxydants, à l'exclusion des masses adhésives hydrocolloïdes contenant un ester d'acide gras de sorbitan éthoxylé.

Selon un mode de réalisation actuellement préféré cette masse adhésive hydrocolloïde comprend :

(a) 2 à 15 parties en poids d'un copolymère acrylate ayant une température de transition vitreuse inférieure à - 20 °C,
(b) 20 à 50 parties en poids d'un dérivé de cellulose notamment la carboxyméthylcellulose de sodium,
(c) 10 à 40 parties en poids d'un mélange formé d'un polyisobutylène de bas poids moléculaire et d'un copolymère séquencé poly(styrène-oléfine-styrène) notamment un poly(styrène-isoprène-styrène),
(d) 20 à 50 parties en poids d'une résine tackifiante,
(e) 2 à 25 parties en poids d'un plastifiant, notamment d'une huile plastifiante,
(f) 0,1 à 2 parties en poids d'au moins un agent antioxydant.

Selon un mode de réalisation particulièrement préféré cette masse adhésive hydrocolloïde comprend :

(a) 2 à 15 parties en poids d'un polymère acrylate ayant une température de transition vitreuse de - 39 °C,
(b) 20 à 50 parties en poids de carboxyméthylcellulose de sodium,
(c) 10 à 35 parties en poids d'un copolymère séquencé poly(styrène-oléfine-styrène) notamment poly(styrène-isoprène-styrène),
(d) 1 à 20 parties en poids d'un polyisobutylène de bas poids moléculaire.

(e) 20 à 50 parties en poids d'une résine tackifiante,

(f) 2 à 25 parties en poids d'une huile plastifiante,

(g) 0,1 à 2 parties d'au moins un agent antioxydant.

Selon un autre mode de réalisation actuellement préféré cette masse adhésive hydrocolloïde comprend :

(a) 2 à 15 parties en poids d'un polymère acrylate ayant une température de transition vitreuse inférieure à - 20 °C,

(b) 20 à 50 parties en poids d'un dérivé de cellulose notamment la carboxyméthylcellulose de sodium,

(c) 5 à 20 parties en poids d'un polymère séquencé poly(styrène-oléfine-styrène) notamment poly(styrène-isoprène-styrène),

(d) 25 à 50 parties en poids d'au moins un polyisobutylène de bas poids moléculaire,

(e) 2 à 20 parties en poids d'un polybutène,

(f) 0,1 à 2 parties en poids d'au moins un antioxydant.

Selon un second aspect, la présente invention a pour objet l'utilisation de ces masses adhésives hydrocolloïdes pour la réalisation de pansements notamment pour le traitement de l'ampoule, des lésions dermo-épidermiques superficielles profondes, chroniques ou aiguës, des plaies exsudatives et des brûlures.

Les composés utilisés pour la réalisation du mélange adhésif des masses adhésives hydrocolloïdes selon l'invention sont ceux habituellement utilisés par l'homme de l'art dans la préparation de masses adhésives et l'on pourra se reporter à cet égard au document de l'état de la technique mentionné précédemment pour les définitions de l'ensemble des composés utilisés ainsi que pour leurs proportions respectives pour obtenir les propriétés adhésives et mécaniques souhaitées.

On pourra ainsi utiliser dans le cadre de la présente invention comme copolymère séquencé du type poly(styrène-oléfine-styrène) des copolymères dans lesquels les séquences oléfines peuvent être constituées d'unités isoprène, butadiène, éthylène-butylène, éthylène-propylène et leurs mélanges.

Parmi ceux-ci, les copolymères triblocs poly(styrène-isoprène-styrène) sont préférés.

Par copolymère tribloc poly(A-B-A) du type poly(styrène-isoprène-styrène) [en abrégé : poly(SIS)] on entend ici un matériau poly(SIS) ayant une teneur en styrène comprise entre 14 et 52 % en poids par rapport au poids dudit poly(SIS). Cette expression couvre aussi des poly(SIS) contenant un mélange de copolymères tribloc poly(SIS) et de copolymères dibloc du type poly(styrène-isoprène).

De tels produits bien connus de l'homme de l'art sont par exemple commercialisés par les sociétés SHELL et EXXON CHEMICAL respectivement sous les dénominations KRATON®D et VECTOR®.

Dans le cadre de la présente invention on préfère les copolymères tribloc ayant une teneur en styrène comprise entre 14 et 30 % en poids par rapport au poids dudit poly(SIS). On préférera en particulier les produits commercialisés respectivement sous les dénominations VECTOR®4114 et VECTOR®4113 par la société EXXON CHEMICAL et KRATON®D-1111CS, KRATON®D-1107 ou KRATON®1161 par la société SHELL CHEMICALS.

Parmi les poly(styrène-butadiène-styrène) on peut citer par exemple le produit commercialisé sous la dénomination KRATON®D-1102 par la société SHELL CHEMICALS.

Comme polyisobutylène, on pourra utiliser dans le cadre de la présente invention des polyisobutylènes de bas poids moléculaire, de l'ordre de 40 000 à 80 000 daltons tels que les composés commercialisés sous la dénomination VISTANEX® par la société EXXON CHEMICAL ou sous la dénomination OPPANOL® par la société BASF.

On préférera en particulier les produits commercialisés sous les dénominations VISTANEX®LM-MS, VISTANEX®LM-MH, OPPANOL®B12 et OPPANOL®B15.

Ces derniers pourront être utilisés seuls ou en mélange.

Divers composés supplémentaires sont en général additionnés à l'association polyisobutylène-poly(styrène-oléfine-styrène) pour réaliser un mélange adhésif qui permette d'obtenir des masses adhésives hydrocolloïdes qui présentent des propriétés d'élasticité, d'adhésion, de stabilité dans le temps et de cohésion optimisées.

Ces deux composés sont ainsi en général associés dans les masses adhésives hydrocolloïdes à des stabilisants tels des antioxydants, des agents permettant d'améliorer l'adhésion tels que des résines dites « tackifiantes, des plastifiants tels que les polybutènes ou les huiles plastifiantes, ou à des agents permettant d'améliorer la cohésion tels que des caoutchoucs butyle ou des polyisobutylènes de haut poids moléculaires, etc.

De telles compositions sont ainsi définies dans « Advances in Pressure Sensitive Adhésive Technology-2-« édité par Donatas Satas en avril 95 dans le chapitre 7 « Wound Dressings » pages 158 à 171 précédemment cité.

De telles formulations sont aussi décrites par exemple dans la demande de brevet EP-A-130061.

On peut ainsi ajouter des polyisobutylènes de haut poids moléculaire, de l'ordre de 400 000 à 2 000 000 daltons comme par exemple les produits commercialisés par la société EXXON CHEMICAL sous les dénominations VISTANEX®L-80 ou VISTANEX®L100.

Parmi les résines tackifiantes qui conviennent pour réaliser ces mélanges adhésifs, on peut ainsi mentionner les résines généralement employées dans le domaine des adhésifs par l'homme de l'art telles les résines polyterpènes ou terpènes modifiées, les résines de colophane hydrogénée, les résines de colophane polymérisée, les résines d'esters de colophane, les résines hydrocarbonées, les mélanges de résines aromatiques et aliphatiques etc. On préférera en particulier dans le cadre de la présente invention une résine de synthèse formée de copolymères en $C_5/C_9$ commercialisée par la société GOOD YEAR sous la dénomination WINGTACK®86.

De même par agents antioxydants on entend les composés couramment employés par l'homme de l'art pour assurer la stabilité vis-à-vis de l'oxygène, la chaleur, l'ozone et les rayonnements ultra violets des composés utilisés dans la formulation des matrices, en particulier les résines tackifiantes et les copolymères séquencés. On peut utiliser un ou plusieurs de ces agents antioxydants en association.

On peut citer comme agents antioxydants appropriés les antioxydants phénoliques comme par exemple les produits commercialisés par la société CIBA-GEIGY sous les dénominations IRGANOX®1010, IRGANOX®565, IRGANOX® 1076 et des antioxydants soufrés comme par exemple le dibutyldithiocarbamate de zinc commercialisé par la société AKZO sous la dénomination PERKACIT®ZDBC.

Dans le cadre de la présente invention, on préférera l'association de l'IRGANOX®1010 et du PERKACIT®ZDBC.

On peut employer tout type de plastifiant habituellement utilisé par l'homme de l'art pour la préparation de masses adhésives hydrocolloïdes à base de copolymère séquencé poly(styrène-oléfine-styrène) ou polyisobutylène. On peut ainsi incorporer dans ces masses adhésives des plastifiants tels que des polybutènes comme par exemple ceux commercialisés par la société BP CHEMICALS sous la dénomination NAPVIS® 10, des huiles plastifiantes ou des dérivés de phtalates tel le dioctylphtalate.

On préférera utiliser des huiles plastifiantes dans le cadre de la présente invention.

Par huile plastifiante on entend ici les huiles minérales ou végétales couramment employées par l'homme de l'art pour plastifier les copolymères séquencés du type styrène-oléfine-styrène ou polyisobutylène utilisés dans la composition des mélanges adhésifs employées dans les masses adhésives hydrocolloïdes.

Les huiles minérales généralement utilisées sont des mélanges de composés de nature paraffinique, naphténique ou aromatique dans des proportions variables.

On peut citer ainsi comme exemple d'huiles plastifiantes les produits commercialisés par la société SHELL sous la dénomination ONDINA® et RISELLA® pour les mélanges à base de composés naphténiques et paraffiniques ou sous la dénomination CATENEX® pour les mélanges à base de composés naphténiques, aromatiques et paraffiniques.

Dans le cadre de la présente invention, on préférera en particulier l'huile plastifiante minérale commercialisée sous la dénomination ONDINA®68.

Par dérivés de cellulose on entend désigner ici les composés de cellulose couramment utilisés par l'homme de l'art dans les masses adhésives hydrocolloïdes, qui présentent une capacité à absorber les liquides hydrophiles et les exsudats et à les transporter rapidement.

Ces dérivés de cellulose sont des polymères de cellulose comme par exemple les hydroxyéthylcelluloses, les hydroxypropylcelluloses, les carboxyméthylcellluloses et leurs sels de métal alcalin tel le sodium ou le calcium. On pourra utiliser ces dérivés de cellulose seuls ou en association.

Dans le cadre de la présente invention on préférera les sels de métal alcalin de carboxyméthylcellulose ; en particulier la carboxyméthylcellulose de sodium. On peut ainsi citer par exemple les carboxyméthylcelluloses de sodium commercialisés sous les dénominations BLANOSE®7H4XF, BLANOSE®7H3XF et AQUASORB®A500 par la société AQUALON.

Les polymères acrylates convenant pour la mise en oeuvre de l'invention sont des composés d'acrylates sensibles à la pression et ayant une température de transition vitreuse (Tg) inférieure à - 20 °C.

De tels composés d'acrylates sont des copolymères formés à partir

- soit d'au moins un monomère, choisi parmi l'ensemble constitué par les esters alkylés de l'acide acrylique dans lesquels le groupe alkyle, linéaire ou ramifié, de l'ester comprend 1 à 18 atomes de carbone, de préférence 4 à 10 atomes de carbone, en particulier 4 à 8 atomes de carbone, comme par exemple les acrylates de méthyle, d'éthyle, de n-propyle, de n-butyle, d'isobutyle, de n-hexyle, de 2-éthylhexyle, de n-octyle, d'isooctyle, de n-décyle et n-dodécyle, associé avec l'acide acrylique;

- soit d'au moins deux monomères, choisis parmi l'ensemble constitué par les esters alkylés de l'acide acrylique dans lesquels le groupe alkyle, linéaire ou ramifié, de l'ester comprend 1 à 18 atomes de carbone de préférence 4 à 10 atomes de carbone, en particulier 4 à 8 atomes de carbone, comme par exemple les acrylates de méthyle, d'éthyle, de n-propyle, de n-butyle, d'isobutyle, de n-hexyle, de 2-éthylhexyle, de n-octyle, d'isooctyle, de n-décyle et n-dodécyle.

Les pourcentages ou proportions respectifs de ces différents monomères sont ajustés de façon à obtenir un copolymère ayant la température de transition vitreuse souhaitée, c'est à dire inférieure à -20 °C.

Dans le cadre de la présente invention, on utilisera de préférence un copolymère contenant au moins un monomère, choisi parmi l'acrylate de n-butyle, l'acrylate de 2-éthylhexyle et l'acrylate d'isooctyle, copolymérisé avec l'acide acrylique.

On préférera tout particulièrement les copolymères contenant de 1 à 20 %, de préférence 1 à 10 %, en poids d'acide acrylique, exprimé par rapport au poids total de l'ensemble des monomères.

De tels composés d'acrylates peuvent aussi être des homopolymères dont le monomère constitutif est choisi dans l'ensemble constitué par les esters alkylés de l'acide acrylique dans lesquels le groupe alkyle de l'ester est soit un groupe alkyle linéaire qui comprend 2 à 12 atomes de carbone soit un groupe isobutyle, 2-éthylhexyle ou isooctyle.

Parmi ces homopolymères on préférera dans le cadre de la présente invention, le polyacrylate de n-butyle.

Selon une caractéristique particulière de l'invention, on choisira les produits bien connus de l'homme de l'art pour être utilisables dans un procédé d'enduction sans solvant connu sous le nom de « hot-melt ».

On peut ainsi citer par exemple les produits commercialisés par la société BASF sous les dénominations suivantes:

- ACRONAL®A150F (homopolymère d'acrylate de n-butyle qui possède une température de transition vitreuse de -41 °C),
- ACRONAL®DS3435X (homopolymère d'acrylate de n-butyle qui possède une température de transition vitreuse de -46 °C),
- ACRONAL®DS3429 (copolymère d'acrylate de n-butyle, d'acrylate de 2-éthylhexyle et d'acide acrylique qui possède une température de transition vitreuse de -31°C), et,
- ACRONAL®DS3458 (copolymère d'acrylate de n-butyle et d'acide acrylique qui possède une température de transition vitreuse de -39 °C.)

On peut citer également le produit commercialisé par la société MONSANTO sous la dénomination :

- MODAFLOW® (copolymère d'acrylate d'éthyle et d'acrylate de 2-éthylhexyle).

Dans le cadre de la présente invention, on préférera tout particulièrement le polymère acrylate commercialisé sous la dénomination ACRONAL®DS3458.

La masse adhésive hydrocolloïde selon l'invention est notamment utile pour toutes les applications à des fins médicales dans lesquelles le produit contenant ladite masse doit être stérilisé. On peut ainsi citer la réalisation de pansements et de bandages pour le traitement de l'ampoule, des lésions dermo-épidermiques superficielles, profondes, chroniques ou aiguës, des plaies exsudatives, des brûlures ainsi que la réalisation de joints adhésifs employés en ostomies.

Dans le cadre de ces applications divers produits à caractère dermatologique, cosmétologique ou thérapeutique peuvent être ajoutés dans la formulation de la masse adhésive hydrocolloïde comme par exemple des antifongiques, des anti-microbiens ou antibactériens tels que la sulfadiazine argentique, des régulateurs de pH, des accélérateurs de cicatrisation, des vitamines, des extraits végétaux, des oligo-éléments, des anesthésiques locaux, des piégeurs d'odeur, du menthol, du salicylate de méthyle, des hormones, des anti-inflammatoires, etc.

Dans le cadre de la réalisation d'un pansement pour le traitement de l'ampoule ou le traitement ou la protection des plaies, de différentes catégories de lésions dermo-épidermiques, brûlures et escarres, on réalise une enduction de la masse adhésive hydrocolloïde selon l'invention sur un support adéquat au grammage souhaité, selon les techniques connues de l'homme de l'art, suivant un procédé en phase solvant ou de préférence suivant un procédé hot-melt, c'est à dire sans solvant, à une température comprise entre 110 et 160 °C.

Le choix du support est réalisé en fonction des propriétés requises (étanchéité, élasticité etc.) suivant le type de pansement et l'application recherchée.

Il peut se présenter sous forme d'un film formé d'une ou plusieurs couches et d'une épaisseur variable de 5 à 150 μm ou d'un non-tissé ou d'une mousse ayant une épaisseur de 10 à 500 μm.

Ces supports à base de matériaux synthétiques ou naturels sont ceux généralement utilisés par l'homme de l'art dans le domaine des pansements et des applications médicales visées ci-dessus.

On peut citer ainsi des mousses en polyéthylène, en polyuréthanne, en PVC ; des non-tissés en polypropylène, polyamide, polyester, éthylcellulose etc.

On préférera cependant utiliser des films en tant que supports et notamment des films en polyuréthanne comme par exemple les produits commercialisés par la société Smith et Nephew sous la référence LASSO ou des films en polyuréthanne réalisés à partir du polyuréthanne commercialisé sous la dénomination ESTANE par la société BF GOODRICH, des films en polyéthylène à basse densité comme par exemple ceux commercialisés par la société SOPAL, des films à base de copolymère thermoplastique de polyéther-polyester comme par exemple les produits commercialisés sous la marque Hytrel® par la société DUPONT DE NEMOURS ou des films complexes réalisés à base de polyuréthanne et d'un non-tissé.

Les pansements réalisés à partir de la masse adhésive hydrocolloïde selon l'invention peuvent se présenter sous n'importe quelle forme géométrique, carrée, rectangulaire, circulaire ou ovale. De même leur taille peut être quelconque et sera adaptée en fonction de la surface de la partie à traiter ou protéger.

De façon pratique, la surface de la masse adhésive hydrocolloïde qui n'est pas liée au support, pourra être recouverte d'une couche ou pellicule de protection pelable avant utilisation du pansement.

L'ensemble ainsi formé pourra être lui-même emballé dans une protection étanche réalisée par exemple au moyen de complexes polyéthylène-aluminium ou dans des blisters.

Les avantages, caractéristiques et applications de l'invention seront mieux compris à la lecture de la description qui va suivre d'exemples de réalisation et d'essais comparatifs.

Bien entendu l'ensemble de ces éléments n'est nullement limitatif mais est donné à titre d'illustration.

Par commodité, dans ce qui suit les abréviations suivantes ont été utilisées :

SIS : copolymère tribloc poly(styrène-isoprène-styrène).

**Exemple 1**

Dans un malaxeur à bras en Z, à une température de l'ordre de 140 °C on introduit successivement 12,5 kg de ONDINA®68 (huile minérale commercialisée par la société SHELL), 14,2 kg de VECTOR®4114 (copolymère SIS commercialisé par la société DEXCO), 3,55 kg de VISTANEX®LM-MH (polymère PIB de bas poids moléculaire commercialisé par la société EXXON CHEMICAL), 0,4 kg de PERKACIT®ZDBC (dibutyl dithiocarbamate de zinc, antioxydant commercialisé par la société AKZO) et 0,4 kg d'IRGANOX®1010 (antioxydant commercialisé par la société CIBA-GEIGY). On malaxe le mélange obtenu entre 120 et 140 °C durant environ 30 minutes. On ajoute ensuite 6,5 kg d'ACRONAL®DS 3458 (copolymère de butylacrylate et d'acide acrylique commercialisé par la société BASF) et on malaxe le mélange obtenu toujours aux alentours de 140 °C durant 40 minutes. On ajoute alors 26,75 kg de WINGTACK® 86 (résine tackifiante commercialisée par la société GOOD YEAR) et on malaxe toujours aux alentours de 140 °C durant 40 minutes. On introduit finalement 35.7 kg de BLANOSE®7H4XF (carboxyméthylcellulose de sodium commercialisé par la société AQUALON) et on malaxe toujours à environ 140 °C durant 40 minutes supplémentaires. On enduit le mélange ainsi obtenu sur une pellicule de papier siliconé à raison de 1000 g/m$^2$ à une température comprise entre 120 et 160 °C. On transfère l'enduction ainsi réalisée sur un support final, de 30 µm d'épaisseur, en polyuréthanne (réalisé à partir de polyuréthanne commercialisé sous la dénomination UCECOAT® par la société UCB). On découpe alors des formes aux dimensions appropriées que l'on conditionne dans des sachets thermosoudables ou des blisters.

**Exemple comparatif 1**

Dans un malaxeur à bras en Z, à une température de l'ordre de 140 °C on introduit successivement 13,95 kg de ONDINA®68, 15,8 kg de VECTOR®4114, 3,95 kg de VISTANEX®LM-MH, 0,4 kg de PERKACIT®ZDBC et 0,4 kg d'IRGANOX®1010. On malaxe le mélange obtenu à environ 140 °C pendant environ 30 minutes. On introduit alors 29,8 kg de WINGTACK®86 et on malaxe toujours à 140 °C durant environ 35 minutes supplémentaires. On introduit finalement 35,7 kg de BLANOSE®7H4XF et on malaxe aux alentours de 140 °C durant encore 45 minutes. On enduit le mélange ainsi obtenu sur une pellicule de papier siliconé à raison de 1000 g/m$^2$ à une température comprise entre 120 et 160 °C. On transfère l'enduction ainsi réalisée sur un support final, de 30 µm d'épaisseur, en polyuréthanne (réalisé à partir de polyuréthanne commercialisé sous la dénomination UCECOAT® par la société UCB). On découpe alors des formes aux dimensions appropriées que l'on conditionne dans des sachets thermosoudables ou des blisters.

**Essais**

Afin de mettre en évidence la résistance à la dégradation de la capacité d'absorption après irradiation des masses adhésives hydrocolloïdes selon l'invention, on a réalisé des mesures d'absorption entre un produit selon l'invention (exemple 1) contenant un polymère acrylate ayant une température de transition vitreuse inférieure à - 20 °C et le même produit sans ce dernier (exemple comparatif 1) avant radiostérilisation β et après radiostérilisation β à différentes doses d'irradiation.

Les mesures d'absorption ont été effectuées suivant le protocole suivant : On utilise un échantillon, réalisé comme décrit dans l'exemple 1 et dans l'exemple comparatif 1, formé du support final, de la masse adhésive hydrocolloïde et de la pellicule de papier siliconé qui sert de protecteur pelable, que l'on découpe de façon à obtenir un ruban adhésif. Pour réaliser la mesure on utilise une cellule de mesure constituée d'un cylindre en aluminium sur lequel on dépose un échantillon de ruban adhésif à tester et sur lequel on vient ensuite fixer un support qui permet de bien solidariser l'ensemble cylindre-échantillon. La partie périphérique de ce support comporte un joint siliconé sur lequel s'adhésive par pression la section périphérique de l'échantillon.

La mesure d'absorption s'effectue par différences de pesées de l'ensemble support-ruban adhésif-cylindre avant et après mise en contact de l'échantillon avec un liquide de référence durant une durée déterminée , ici 24 heures.

Dans les tests suivants le liquide de référence est une solution de Dextran D4876 (commercialisé par la société Sigma) à 60 g par litres dans une solution de chlorure de sodium 0,15 molaire.

Les mesures sont réalisées de la façon suivante :

1) on découpe un échantillon (ici par exemple de 16 cm$^2$) du ruban adhésif à tester et on enlève le film protecteur ;

2) on incorpore l'échantillon dans la cellule de mesure comme décrit ci-dessus ;

3) l'ensemble ainsi obtenu est pesé ; soit $P_0$ le poids obtenu ;

4) on introduit alors 20 ml du liquide de référence précédemment préparé dans le cylindre ;

5) on laisse l'ensemble en contact à 23 °C pendant 24 heures ;

6) à l'issue de ces 24 heures on pèse à nouveau, après élimination de la solution non absorbée, l'ensemble support-échantillon-cylindre, soit $P_1$ le poids obtenu ;

7) on calcule le pouvoir absorbant qui correspond à l'absorption surfacique grâce à la formule : Absorption $=4(P_1-P_0)/\pi D^2$ où D représente le diamètre du cylindre soit ici 0,0357 m.

D'où l'absorption exprimée en $g/m^2$ et définie ici par :

$$\text{Absorption} = (P_1 - P_0)10^3$$

Pour chaque test on réalise au moins 5 essais.

Le pouvoir absorbant obtenu est la moyenne de ces différents essais.

La stérilisation par rayonnement $\beta$ s'effectue de façon classique. Les produits à stériliser défilent sur un tapis roulant et en agissant sur la vitesse du convoyeur on règle la dose de traitement envoyée.

Le produit de l'exemple 1 et de l'exemple comparatif 1 ont été ainsi traités aux doses suivantes 15, 25, 35 et 45 kilograys.

L'ensemble des résultats d'absorption pour ces différentes doses sont rassemblées dans le tableau I dans lequel :

A(EX1) et A(EC1) représentent respectivement les absorptions à 24 heures exprimées en $g/m^2$ des rubans adhésifs obtenus selon l'exemple 1 et selon l'exemple comparatif 1 et,

R, exprimé en pourcentage, représente pour chaque dose d'irradiation le rapport entre l'absorption d'un ruban adhésif stérilisé et l'absorption du même ruban adhésif non stérilisé.

**TABLEAU I**

|  | Non stérilisé | 15 KGY | 25 KGY | 35 KGY | 45 KGY |
|---|---|---|---|---|---|
| A(EX1) | 5590 | 4880 | 4520 | 4260 | 3950 |
| R | - | 87,3 | 80,8 | 72,2 | 70,7 |
| A(EC1) | 1820 | 620 | 430 | 460 | 400 |
| R | - | 34 | 23,6 | 25,2 | 22 |

L'analyse du tableau I illustre parfaitement l'intérêt d'utiliser, dans une masse adhésive hydrocolloïde formée d'un mélange adhésif à base de polyisobutylène et d'un copolymère séquencé poly(styrène-oléfine-styrène), dans ce cas un poly(styrène-isoprène-styrène), et d'un dérivé de cellulose, dans ce cas la carboxyméthylcellulose de sodium, un copolymère acrylate ayant une température de transition vitreuse inférieure à - 20 °C pour augmenter la résistance à la dégradation de la capacité d'absorption de la masse adhésive hydrocolloïde après la radiostérilisation.

On constate en effet que, déjà à une dose d'irradiation de 15 kilograys inférieure à la dose de 25 kilograys utilisée généralement pour stériliser les produits avec une marge de sécurité , la valeur R qui représente le pourcentage d'absorption résiduelle par rapport à la masse adhésive non stérilisée est de 87,3 % pour la masse adhésive hydrocolloïde contenant le polymère acrylate (exemple 1) contre seulement 34 % en l'absence de ce dernier (exemple comparatif 1)

De la même façon ce rapport est de 80,8 % contre 23,6 % à 25 kilograys, de 72,2 % contre 25,2 % à 35 kilograys et de 70,7 % contre 22 % à 45 kilograys.

Sachant qu'en pratique lors de la radiostérilisation d'un lot industriel un produit reçoit une dose qui varie dans une plage comprise entre 25 et 45 kilograys, on comprend ainsi l'intérêt de la présente invention, puisque l'ajout d'un polymère acrylate dans la masse adhésive hydrocolloïde permet de conserver les ¾ de l'absorption initiale contre seulement ¼ pour le même produit sans polymère acrylate, dans cette plage de doses.

Ce résultat constitue une avancée significative dans le domaine des masses adhésives dans lesquelles l'hydrocolloïde est un dérivé de cellulose. On obtient en effet ainsi un produit commercial qui présente un bon niveau d'absorption sans nécessiter l'utilisation de mélanges avec des hydrocolloïdes d'autres natures comme par exemple les gommes ou la pectine ou la gélatine.

Ceci est d'autant plus important que l'utilisation de composés d'origine animale tels que la gélatine, pourrait devenir problématique, notamment dans les produits pharmaceutiques. La présente invention permet ainsi de réaliser des masses adhésives hydrocolloïdes à base de polyisobutylène et de poly(styrène-oléfine-styrène) plus simples dans lesquelles on diminue les risques d'incompatibilité entre les composés et où il est donc plus facile d'optimiser les propriétés adhésives cohésives et d'absorption.

**Revendications**

1. Masse adhésive hydrocolloïde notamment utilisable à des fins médicales **caractérisée en ce qu'**elle comprend :

(a) 2 à 15 parties en poids d'un polymère acrylate ayant une température de transition vitreuse inférieure à - 20 °C,
(b) 20 à 50 parties en poids d'au moins un dérivé de cellulose,
(c) 32 à 120 parties en poids d'un mélange adhésif constitué d'au moins un polyisobutylène de bas poids moléculaire et d'un polymère séquencé poly(styrène-oléfine-styrène) auxquels sont associés un ou plusieurs composés choisis parmi les polyisobutylènes de haut poids moléculaire, les polybutènes, les résines poisseuses dites tackifiantes, les caoutchoucs butyle, les plastifiants et les antioxydants, à l'exclusion des masses adhésives hydrocolloïdes contenant un ester d'acide gras de sorbitan éthoxylé.

2. Masse adhésive hydrocolloïde selon la revendication 1 **caractérisée en ce que** le polymère acrylate ayant une température de transition vitreuse inférieure à - 20 °C est un copolymère formé à partir d'au moins un monomère, choisi dans l'ensemble constitué par les esters alkylés de l'acide acrylique dans lesquels le groupe alkyle, linéaire ou ramifié, de l'ester comprend 1 à 18 atomes de carbone de préférence 4 à 10 atomes de carbone, en particulier 4 à 8 atomes de carbone, comme par exemple les acrylates de méthyle, d'éthyle, de n-propyle, de n-butyle, d'isobutyle, de n-hexyle, de 2-éthylhexyle, de n-oxtyle, d'isooctyle, de n-décyle et n-dodécyle, copolymérisé avec l'acide acrylique.

3. Masse adhésive hydrocolloïde selon la revendication 2, **caractérisée en ce que** le copolymère acrylate précité est un copolymère formé à partir d'au moins un monomère, choisi dans l'ensemble constitué par l'acrylate de n-butyle, l'acrylate de 2-éthylhexyle et l'acrylate d'isooctyle, copolymérisé avec l'acide acrylique et de préférence un copolymère de n-butylacrylate et d'acide acrylique ayant une température de transition vitreuse de - 39 °C ou de n-butylacrylate, 2-éthylhexyle acrylate et d'acide acrylique ayant une température de transition vitreuse de - 31 °C.

4. Masse adhésive hydrocolloïde selon la revendication 3, **caractérisée en ce que** le copolymère acrylate précité comprend de 1 à 20 %, de préférence 1 à 10 %, en poids d'acide acrylique exprimé par rapport au poids total de l'ensemble des monomères.

5. Masse adhésive hydrocolloïde selon la revendication 1 **caractérisée en ce que** le polymère acrylate ayant une température de transition vitreuse inférieure à - 20 °C est un copolymère formé à partir d'au moins deux monomères, choisis dans l'ensemble constitué par les esters alkylés de l'acide acrylique dans lesquels le groupe alkyle, linéaire ou ramifié, de l'ester comprend 1 à 18 atomes de carbone, de préférence 4 à 10 atomes de carbone, en particulier 4 à 8 atomes de carbone, comme par exemple les acrylates de méthyle, d'éthyle, de n-propyle, de n-butyle, d'isobutyle, de n-hexyle, de 2-éthylhexyle, de n-octyle, d'isooctyle, de n-décyle et n-dodécyle.

6.  Masse adhésive hydrocolloïde selon la revendication 1 **caractérisée en ce que** le polymère acrylate ayant une température de transition vitreuse inférieure à - 20 °C est un homopolymère dont le monomère constitutif est choisi dans l'ensemble constitué par les esters alkylés de l'acide acrylique dans lesquels le groupe alkyle de l'ester est soit un groupe alkyle linéaire qui comprend 2 à 12 atomes de carbone, soit un groupe isobutyle, 2-éthylhexyle ou isooctyle et de préférence un homopolymère d'acrylate de n-butyle ayant une température de transition vitreuse de - 41 °C.

7.  Masse adhésive hydrocolloïde selon l'une des revendications 1 à 6 **caractérisée en ce qu'**elle comprend :

    (a) 2 à 15 parties en poids d'un copolymère acrylate ayant une température de transition vitreuse inférieure à - 20 °C,
    (b) 20 à 50 parties en poids d'un dérivé de cellulose notamment la carboxyméthylcellulose de sodium,
    (c) 10 à 40 parties en poids d'un mélange formé d'un polyisobutylène de bas poids moléculaire et d'un copolymère séquencé poly(styrène-oléfine-styrène) notamment poly(styrène-isoprène-styrène),
    (d) 20 à 50 parties en poids d'une résine tackifiante,
    (e) 2 à 25 parties en poids d'un plastifiant, notamment d'une huile plastifiante,
    (f) 0,1 à 2 parties en poids d'au moins un agent antioxydant.

8.  Masse adhésive hydrocolloïde selon la revendication 7 **caractérisée en ce que** le plastifiant précité est une huile plastifiante minérale et de préférence une huile constituée de composés naphténiques, paraffiniques et aromatiques.

9.  Masse adhésive hydrocolloïde selon l'une des revendications 7 ou 8, **caractérisée en ce qu'**elle comprend :

    (a) 2 à 15 parties en poids d'un polymère acrylate ayant une température de transition vitreuse de - 39 °C,
    (b) 20 à 50 parties en poids de carboxyméthylcellulose de sodium,
    ($c_1$) 10 à 35 parties en poids d'un copolymère séquencé poly(styrène-oléfine-styrène) notamment poly(styrène-isoprène-styrène),
    ($c_2$) 1 à 20 parties en poids d'un polyisobutylène de bas poids moléculaire,
    (d) 20 à 50 parties en poids d'une résine tackifiante,
    (e) 2 à 25 parties en poids d'une huile plastifiante,
    (f) 0, 1 à 2 parties d'au moins un agent antioxydant.

10. Masse adhésive hydrocolloïde selon l'une des revendications 1 à 6 **caractérisée en ce qu'**elle comprend :

    (a) 2 à 15 parties en poids d'un polymère acrylate ayant une température de transition vitreuse inférieure à - 20 °C,
    (b) 20 à 50 parties en poids d'un dérivé de cellulose notamment la carboxyméthylcellulose de sodium,
    (c) 5 à 20 parties en poids d'un polymère séquencé poly(styrène-oléfine-styrène) notamment poly(styrène-isoprène-styrène),
    (d) 25 à 50 parties en poids d'au moins un polyisobutylène de bas poids moléculaire,
    (e) 2 à 20 parties en poids d'un polybutène,
    (f) 0,1 à 2 parties en poids d'au moins un antioxydant.

11. Masse adhésive hydrocolloïde selon l'une des revendications 1 à 10, **caractérisée en ce que** le copolymère séquencé précité est un poly(styrène-isoprène-styrène) qui présente une teneur en styrène comprise entre 14 et 52 % en poids par rapport au poids dudit copolymère, et de préférence une teneur comprise entre 14 et 30 % en poids.

12. Masse adhésive hydrocolloïde selon l'une des revendications 1 à 10, **caractérisée en ce que** la matrice adhésive de ladite masse adhésive hydrocolloïde comprend un ou plusieurs polyisobutylènes de bas poids moléculaire compris entre 40 000 et 80 000 daltons.

13. Masse adhésive hydrocolloïde selon l'une des revendications 1 à 12, **caractérisée en ce que** le dérivé de cellulose est un sel de métal alcalin de carboxyméthylcellulose, et de préférence la carboxyméthylcellulose de sodium.

14. Utilisation d'une masse adhésive hydrocolloïde selon l'une quelconque des revendications 1 à 13 pour la préparation d'un pansement destiné au traitement de l'ampoule, des lésions dermo-épidermiques superficielles profondes, chroniques ou aiguës, exsudatives ou brûlures, formé d'un support sur lequel est enduite ladite masse adhésive hydrocolloïde et éventuellement d'une pellicule de protection pelable.

**Patentansprüche**

1. Hydrokolloidkleber, insbesondere zu medizinischen Zwecken, **dadurch gekennzeichnet, dass** dieser aufweist:

   (a) 2 bis 15 Gewichtsanteile eines Acrylatpolymers mit einer Glasübergangstemperatur kleiner als -20°C,

   (b) 20 bis 50 Gewichtsanteile wenigstens eines Zellulosederivats,

   (c) 32 bis 120 Gewichtsanteile einer Klebermischung, welche gebildet ist aus wenigstens einem Polyisobutylen mit geringem Molekulargewicht und einem sequenzierten Polymer Poly(styrol-olefin-styrol), mit welchen ein oder mehrere Verbindungen verbunden sind, die gewählt sind aus Polyisobutylenen mit hohem Molekulargewicht, den Polybutenen, klebrigen Harzen, genannt Klebrigmacher, den Butylkautschuken, den Weichmachern und den Antioxidantien, unter Ausschluss von Hydrokolloidklebern, welche einen Fettsäureester von ethoxyliertem Sorbitan beinhalten.

2. Hydrokolloidkleber gemäß Anspruch 1, **dadurch gekennzeichnet, dass** das Acrylatpolymer, welches eine Glasübergangstemperatur kleiner als -20°C aufweist, ein Copolymer ist, gebildet aus wenigstens einem Monomer, in dem gebildeten Komplex gewählt aus den alkylierten Estern der Acrylsäure, in welchen die Alkylgruppe, linear oder verzweigt, des Esters 1 bis 18 Kohlenstoffatome aufweist, vorzugsweise 4 bis 10 Kohlenstoffatome, insbesondere 4 bis 8 Kohlenstoffatome, wie zum Beispiel die Acrylate von Methyl, Ethyl, n-Propyl, n-Butyl, Isobutyl, n-Hexyl, 2-Ethylhexyl, n-Octyl, Isooctyl, n-Decyl und n-Dodecyl, copolymerisiert mit der Acrylsäure.

3. Hydrokolloidkleber gemäß Anspruch 2, **dadurch gekennzeichnet, dass** das oben genannte Acrylatcopolymer ein Copolymer ist, gebildet aus wenigstens einem Monomer, in dem gebildeten Komplex gewählt aus dem Acrylat von n-Butyl, dem Acrylat von 2-Ethylhexyl und dem Acrylat von Isooctyl, copolymerisiert mit der Acrylsäure, und vorzugsweise ein Copolymer von n-Butylacrylat und Acrylsäure mit einer Glasübergangstemperatur von -39°C oder von n-Butylacrylat, 2-Ethylhexylacrylat und Acrylsäure mit einer Glasübergangstemperatur von -31°C

4. Hydrokolloidkleber gemäß Anspruch 3, **dadurch gekennzeichnet, dass** das oben genannte Acrylatcopolymer 1 bis 20 Gew.-% vorzugsweise 1 bis 10 Gew.-% Acrylsäure aufweist, ausgedrückt in Bezug auf das Gesamtgewicht des Komplexes von Monomeren.

5. Hydrokolloidkleber gemäß Anspruch 1, **dadurch gekennzeichnet, dass** das Acrylatpolymer mit einer Glasübergangstemperatur kleiner als -20°C ein Copolymer ist, gebildet aus wenigstens zwei Monomeren, in dem gebildeten Komplex gewählt aus den alkylierten Estern von Acrylsäure, in welchen die Alkylgruppe, linear oder verzweigt, des Esters 1 bis 18 Kohlenstoffatome aufweist, vorzugsweise 4 bis 10 Kohlenstoffatome, insbesondere 4 bis 8 Kohlenstoffatome, wie zum Beispiel die Acrylate von Methyl, Ethyl, n-Propyl, n-Butyl, Isobutyl, n-Hexyl, 2-Ethylhexyl, n-Octyl, Isooctyl, n-Decyl und n-Dodecyl.

6. Hydrokolloidkleber gemäß Anspruch 1, **dadurch gekennzeichnet, dass** das Acrylatpolymer mit einer Glasübergangstemperatur kleiner als -20°C ein Homopolymer ist, dessen Bestandteil bildendes Monomer in dem gebildeten Komplex ge-wählt ist aus den alkylierten Estern von Acrylsäure, in denen die Alkylgruppe des Esters entweder eine lineare Alkylgruppe ist, die 2 bis 12 Kohlenstoffatome aufweist, oder eine Isobutyl-, 2-Ethylhexyl- oder Isooctyl-Gruppe ist, und vorzugsweise ein Homopolymer des Acrylats von n-Butyl mit einer Glasübergangstemperatur von -41°C.

7. Hydrokolloidkleber gemäß einem der Ansprüche 1 bis 6, **dadurch gekennzeichnet, dass** dieser aufweist:

   (a) 2 bis 15 Gewichtsanteile eines Acrylatcopolymers mit einer Glasübergangstemperatur kleiner als 20°C,

   (b) 20 bis 50 Gewichtsanteile eines Zellulosederivats, insbesondere Carboxymethylzellulose von Natrium,

   (c) 10 bis 40 Gewichtsanteile einer Mischung, gebildet aus einem Polyisobotylen mit niedrigem Molekulargewicht und einem sequenzierten Copolymer Poly(styrol-olefin-styrol), insbesondere Poly(styrol-isopren-styrol),

   (d) 20 bis 50 Gewichtsanteile eines Klebrigmacher-Harzes,

(e) 2 bis 25 Gewichtsanteile eines Weichmachers, insbesondere eines Weichmacheröls,

(f) 0,1 bis 2 Gewichtsanteile wenigstens eines Antioxidants.

8. Hydrokolloidkleber gemäß Anspruch 7, **dadurch gekennzeichnet, dass** der oben genannte Weichmacher ein mineralisches Weichmacheröl ist, und vorzugsweise ein Öl gebildet aus naphthenischen, paraffinischen und aromatischen Verbindungen.

9. Hydrokolloidkleber gemäß einem der Ansprüche 7 oder 8, **dadurch gekennzeichnet, dass** dieser aufweist:

(a) 2 bis 15 Gewichtsanteile eines Acrylatpolymers mit einer Glasübergangstemperatur von -39°C,

(b) 20 bis 50 Gewichtsanteile von Carboxymethylzellulose von Natrium,

($c_1$) 10 bis 35 Gewichtsanteile eines sequenzierten Copolymers Poly(styrololefin-styrol), insbesondere Poly(styrol-isopren-styrol),

($c_2$) 1 bis 20 Gewichtsanteile eines Polyisobutylens mit niedrigem Molekulargewicht,

(d) 20 bis 50 Gewichtsanteile eines Klebrigmachers-Harzes,

(e) 2 bis 25 Gewichtsanteile eines Weichmacheröls,

(f) 0,1 bis 2 Anteile wenigstens eines Antioxidants.

10. Hydrokolloidkleber gemäß einem der Ansprüche 1 bis 6, **dadurch gekennzeichnet, dass** dieser aufweist:

(a) 2 bis 15 Gewichtsanteile eines Acrylatpolymers mit einer Glasübergangstemperatur kleiner als -20°C,

(b) 20 bis 50 Gewichtsanteile eines Zellulosederivats, insbesondere von Carboxymethylzellulose von Natrium,

(c) 5 bis 20 Gewichtsanteile eines sequenzierten Polymers Poly(styrol-olefinstyrol), insbesondere Poly(styrol-isopren-styrol),

(d) 25 bis 50 Gewichtsanteile wenigstens eines Polyisobutylens mit niedrigem Molekulargewicht,

(e) 2 bis 20 Gewichtsanteile eines Polybutens,

(f) 0,1 bis 2 Gewichtsanteile wenigstens eines Antioxidants.

11. Hydrokolloidkleber gemäß einem der Ansprüche 1 bis 10, **dadurch gekennzeichnet, dass** das oben genannte sequenzierte Copolymer ein Poly(styrolisoprenstyrol) ist, welches einen Styrolgehalt zwischen 14 und 52 Gew.-% im Verhältnis zu dem Gewicht des Copolymers aufweist, und vorzugsweise einen Gehalt zwischen 14 und 30 Gew.-%.

12. Hydrokolloidkleber gemäß einem der Ansprüche 1 bis 10, **dadurch gekennzeichnet, dass** die Klebermatrix des Hydrokolloidklebers ein oder mehrere Polyisobutylene mit niedrigem Molekulargewicht zwischen 40 000 und 80 000 Daltons aufweist.

13. Hydrokolloidkleber gemäß einem der Ansprüche 1 bis 12, **dadurch gekennzeichnet, dass** das Zellulosederivat ein Alkalimetallsalz von Carboxymethylzellulose ist, und vorzugsweise von Carboxymethylzellulose von Natrium.

14. Verwendung eines Hydrokolloidklebers gemäß irgend einem der Ansprüche 1 bis 13 für die Herstellung eines Pflasters, welches für die Behandlung von Blasen, tiefen dermo-epidermischen Oberflächenverletzungen, chronisch oder akut, exsudativ, oder Brandwunden vorgesehen ist, gebildet aus einem Träger, auf welchen der Hydrokolloidkleber aufgestrichen wird, und gegebenenfalls aus einem abziehbaren Schutzfilm.

**Claims**

1. Hydrocolloid adhesive mass useful especially for medical purposes, **characterized in that** it comprises:

(a) 2 to 15 parts by weight of an acrylate polymer with a glass transition temperature below -20°C;
(b) 20 to 50 parts by weight of at least one cellulose derivative; and
(c) 32 to 120 parts by weight of an adhesive mixture consisting of at least one low molecular polyisobutylene and a poly(styrene/olefin/styrene) block polymer, with which are associated one or more compounds selected from high molecular polyisobutylenes, polybutenes, sticky or "tackifying" resins, butyl rubbers, plasticizers and antioxidants, except for hydrocolloid adhesive mass containing an ethoxylated sorbitan fatty ester.

**2.** Hydrocolloid adhesive mass according to claim 1, **characterized in that** the acrylate polymer with a glass transition temperature below -20°C is a copolymer formed of at least one monomer selected from the group consisting of acrylic acid alkyl esters in which the linear or branched alkyl group of the ester contains 1 to 18 carbon atoms, preferably 4 to 10 carbon atoms and particularly 4 to 8 carbon atoms, for example methyl, ethyl, n-propyl, n-butyl, isobutyl, n-hexyl, 2-ethylhexyl, n-octyl, isooctyl, n-decyl and n-dodecyl acrylates, copolymerized with acrylic acid.

**3.** Hydrocolloid adhesive mass according to claim 2, **characterized in that** the above-mentioned acrylate copolymer is a copolymer formed of at least one monomer selected from the group consisting of n-butyl acrylate, 2-ethylhexyl acrylate and isooctyl acrylate, copolymerized with acrylic acid, and preferably an n-butyl acrylate/acrylic acid copolymer with a glass transition temperature of -39°C or an n-butyl acrylate/2-ethylhexyl acrylate/acrylic acid copolymer with a glass transition temperature of -31°C.

**4.** Hydrocolloid adhesive mass according to claim 3, **characterized in that** the above-mentioned acrylate copolymer comprises from 1 to 20% and preferably 1 to 10% by weight of acrylic acid, expressed relative to the total weight of all the monomers.

**5.** Hydrocolloid adhesive mass according to claim 1, **characterized in that** the acrylate polymer with a glass transition temperature below -20°C is a copolymer formed of at least two monomers selected from the group consisting of acrylic acid alkyl esters in which the linear or branched alkyl group of the ester contains 1 to 18 carbon atoms, preferably 4 to 10 carbon atoms and particularly 4 to 8 carbon atoms, for example methyl, ethyl, n-propyl, n-butyl, isobutyl, n-hexyl, 2-ethylhexyl, n-octyl, isooctyl, n-decyl and n-dodecyl acrylates.

**6.** Hydrocolloid adhesive mass according to claim 1, **characterized in that** the acrylate polymer with a glass transition temperature below -20°C is a homopolymer whose constituent monomer is selected from the group consisting of acrylic acid alkyl esters in which the alkyl group of the ester is either a linear alkyl group containing 2 to 12 carbon atoms or an isobutyl, 2-ethylhexyl or isooctyl group, and preferably an n-butyl acrylate homopolymer with a glass transition temperature of -41°C.

**7.** Hydrocolloid adhesive mass according to one of claims 1 to 6, **characterized in that** it comprises:

(a) 2 to 15 parts by weight of an acrylate copolymer with a glass transition temperature below -20°C;
(b) 20 to 50 parts by weight of a cellulose derivative, especially sodium carboxymethylcellulose;
(c) 10 to 40 parts by weight of a mixture formed of a low molecular polyisobutylene and a poly(styrene/olefin/styrene) block copolymer, especially a poly(styrene/isoprene/styrene);
(d) 20 to 50 parts by weight of a tackifying resin;
(e) 2 to 25 parts by weight of a plasticizer, especially a plasticizing oil; and
(f) 0.1 to 2 parts by weight of at least one antioxidant.

**8.** Hydrocolloid adhesive mass according to claim 7, **characterized in that** the above-mentioned plasticizer is a mineral plasticizing oil and preferably an oil consisting of naphthenic, paraffinic and aromatic compounds.

**9.** Hydrocolloid adhesive mass according to claim 7 or 8, **characterized in that** it comprises:

(a) 2 to 15 parts by weight of an acrylate polymer with a glass transition temperature of -39°C;
(b) 20 to 50 parts by weight of sodium carboxymethylcellulose;
($c_1$) 10 to 35 parts by weight of a poly(styrene/olefin/styrene) block copolymer, especially a poly(styrene/isoprene/styrene);
($c_2$) 1 to 20 parts by weight of a low molecular polyisobutylene;
(d) 20 to 50 parts by weight of a tackifying resin;
(e) 2 to 25 parts by weight of a plasticizing oil; and
(f) 0.1 to 2 parts of at least one antioxidant.

**10.** Hydrocolloid adhesive mass according to one of claims 1 to 6, **characterized in that** it comprises:

(a) 2 to 15 parts by weight of an acrylate polymer with a glass transition temperature below -20°C;
(b) 20 to 50 parts by weight of a cellulose derivative, especially sodium carboxymethylcellulose;
(c) 5 to 20 parts by weight of a poly(styrene/olefin/styrene) block polymer, especially a poly(styrene/isoprene/styrene);
(d) 25 to 50 parts by weight of at least one low molecular polyisobutylene;
(e) 2 to 20 parts by weight of a polybutene; and

(t) 0.1 to 2 parts by weight of at least one antioxidant.

**11.** Hydrocolloid adhesive mass according to one of claims 1 to 10, **characterized in that** the above-mentioned block copolymer is a poly(styrene/isoprene/styrene) with a styrene content of between 14 and 52% by weight, based on the weight of said copolymer, and preferably with a content of between 14 and 30% by weight.

**12.** Hydrocolloid adhesive mass according to one of claims 1 to 10, **characterized in that** the adhesive matrix of said hydrocolloid adhesive mass comprises one or more polyisobutylenes with a low molecular weight of between 40,000 and 80,000 daltons.

**13.** Hydrocolloid adhesive mass according to one of claims 1 to 12, **characterized in that** the cellulose derivative is an alkali metal salt of carboxymethyl cellulose, preferably sodium carboxymethylcellulose.

**14.** Use of a hydrocolloid adhesive mass according to any one of claims 1 to 13 for the preparation of a dressing for the treatment of blisters, superficial, deep, chronic or acute dermo-epidermal lesions, exudative or bums, said dressing being formed of a support onto which said hydrocolloid adhesive mass is coated, and optionally of a peel-off protective film.